(19) Europäisches Patentamt
European Patent Office
Office européen des brevets

(11) Veröffentlichungsnummer: **0 037 590**
B1

(12) # EUROPÄISCHE PATENTSCHRIFT

(45) Veröffentlichungstag der Patentschrift: **19.06.85**

(51) Int. Cl.[4]: **C 08 F 20/60**, C 02 F 1/56

(21) Anmeldenummer: **81104251.4**

(22) Anmeldetag: **21.12.79**

(60) Veröffentlichungsnummer der früheren Anmeldung nach Art. 76 EPÜ:

(54) **Polymerisate von quartäre Gruppen enthaltenden Acrylyl- bzw. Methacrylylharnstoffen und ihre Verwendung als Flockungs-, Sedimentations-, Entwässerungs- und Retentionshilfsmittel.**

(30) Priorität: **27.12.78 DE 2856384**

(43) Veröffentlichungstag der Anmeldung: **14.10.81 Patentblatt 81/41**

(45) Bekanntmachung des Hinweises auf die Patenterteilung: **19.06.85 Patentblatt 85/25**

(84) Benannte Vertragsstaaten: **AT BE CH DE FR GB IT LU NL SE**

(56) Entgegenhaltungen:
**DE - A - 888 316**
**FR - A - 2 259 816**

(73) Patentinhaber: **Chemische Fabrik Stockhausen GmbH, Bäkerpfad 25, D-4150 Krefeld (DE)**

(72) Erfinder: **Küster, Erich, Dipl.-Chem., Dr., Alexanderplatz 15, D-4150 Krefeld (DE)**
Erfinder: **Dahmen, Kurt, Dipl.-Chem., Dr., Von-Velsen-Strasse 6, D-4050 Mönchengladbach 2 (DE)**
Erfinder: **Barthell, Eduard, Dipl.-Chem., Dr., Minkweg 18a, D-4150 Krefeld (DE)**

(74) Vertreter: **Klöpsch, Gerald, Dr.-Ing., An Gross St. Martin 6, D-5000 Köln 1 (DE)**

EP 0 037 590 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft Polymerisate von Acrylyl- bzw. Methacrylylharnstoffen, die quartäre Gruppen enthalten sowie ihre Verwendung als Flockungs-, Sedimentations-, Entwässerungs- und Retentionshilfsmittel.

Unter Polymerisaten werden Homopolymerisate, Copolymerisate, Terpolymerisate und andere Interpolymerisate verstanden.

Die erfindungsgemäßen Polymerisate sind durch Struktureinheiten (wiederkehrende Einheiten) der Formel

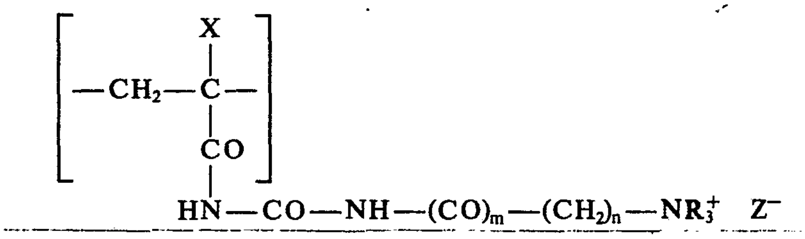

worin X entweder Wasserstoff oder eine Methylgruppe, R ein niederer Acrylrest mit 1 bis 4 Kohlenstoffatomen, m entweder 0 oder 1 und n eine ganze Zahl zwischen 1 und 4 bedeutet und Z für ein beliebiges salzbildendes Anion, vorzugsweise ein Halogenidion steht, gekennzeichnet. Diese Struktureinheiten leiten sich von Harnstoffen der allgemeinen Formel ab

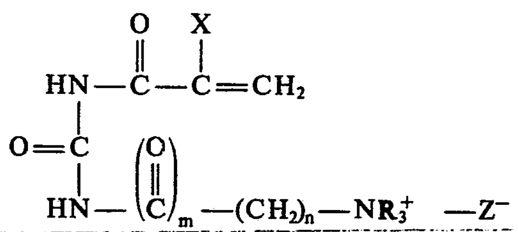

(I)

in der X, R, m, n und Z die erwähnte Bedeutung haben.

Als geeignete Comonomere kommen alle wasserlöslichen, olefinisch ungesättigten Verbindungen in Frage, beispielsweise Acryl- und Methacrylsäureamid und deren N-substituierte Derivate, insbesondere N-[3-Dimethylaminopropyl]-acrylamid sowie dessen Salze und quartäre Verbindungen, N-Methylolacrylamid und -methacrylamid sowie deren Äther, aminosubstituierte Acryl- und Methacrylsäureester, wie z. B. Diäthylaminoäthylacrylat und Dimethylaminoäthylmethacrylat sowie deren Salze und quartäre Verbindungen, weiterhin Vinylester, wie z. B. Vinylacetat.

Die Polymerisation erfolgt nach bekannten Verfahren:

Sie kann thermisch, photochemisch, mit Strahlen oder mit den üblichen radikalischen Initiatoren ausgelöst werden. Hierzu kann die Polymerisation in Lösung, Suspension oder Emulsion durchgeführt werden. Geeignete Initiatoren sind u. a. anorganische Peroxide, wie z. B. tert.-Butyl-, Kumolhydroperoxid oder Dibenzoylperoxid, in Radikale zerfallende aliphatische Azoverbindungen, wie z. B. 2,2'-Azo-bis-isobuttersäurenitril, Redoxkatalysatorsysteme wie die Systeme Persulfat oder Chlorat mit Disulfit oder Eisen(II)-salzen, ferner die als Radikalbildner bekannten Chelate von Übergangsmetallen. Die Initiatoren werden im allgemeinen in einer Menge von 0,001 bis 1 Gew.-%, bezogen auf die Monomermenge, verwendet. Die optimale Menge und der optimal wirksame Initiator lassen sich durch Versuche leicht ermitteln.

Die Polymerisation wird vorteilhaft in Gegenwart eines Lösungs- oder Verdünnungsmittels durchgeführt. Die bei anderen Monomeren üblichen Suspensions-, Lösungs- oder Emulsionspolymerisationsverfahren sind auch für die erfindungsgemäßen Polymerisate geeignet. Gegebenenfalls können Hilfsmittel, wie Puffersubstanzen, Dispergiermittel, Schutzkolloide und dergleichen verwendet werden. Die erfindungsgemäßen Polymerisate können als Flockungs-, Sedimentations-, Entwässerungs- und Retentionsmittel eingesetzt werden. Sie zeichnen sich durch Löslichkeit in Wasser aus. Weiterhin weisen diese wäßrigen Lösungen nur geringe Viskositäten auf, was für die Anwendung sehr vorteilhaft ist. Wegen des Vorhandenseins der sehr stabilen Harnstoffgruppierungen in den Polymeren ist auch die Hydrolysebeständigkeit ausgezeichnet.

Die als Ausgangsmonomere verwendeten Harnstoffe der allgemeinen Formel (I) werden nach einem Verfahren, das nicht Gegenstand der Erfindung ist, hergestellt, indem man Halogenalkyl- bzw. Halogenacylisocyanate der Formel

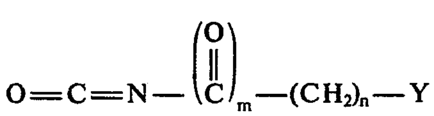

(III)

mit $\alpha,\beta$-ungesättigten Carbonsäureamiden der Formel

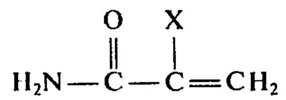

(IV)

zu halogensubstituierten Harnstoffen der allgemeinen Formel

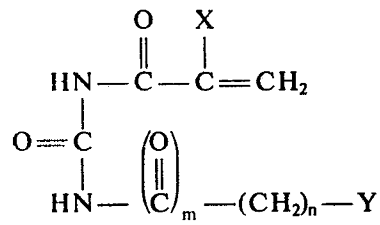

(II)

umsetzt, wobei in den vorstehenden Formeln X, m und n die im Anspruch 1 angegebene Bedeutung haben und Y ein Halogenatom, vorzugsweise Chlor, Brom oder Jod ist, worauf man die halogensubstituierten Harnstoffe der allgemeinen Formel II mit einem Trialkylamin der Formel $NR_3$, in der R ein niederer Alkylrest mit 1 bis 4 Kohlenstoffatomen — auf an sich bekannte Weise — bei Temperaturen von etwa 20 bis 100°C und einen Druck von 1 bis 4 bar in einem Lösungsmittel zur quartären Verbindung I umsetzt.

Die Erfindung wird durch die nachfolgenden Beispiele erläutert.

## A) Herstellung der Ausgangsmonomeren (Harnstoffe der Formel I)

### Beispiel 1

### Acrylyl-ureylen-äthylen-trimethylammoniumchlorid

Man kühlt 35,3 g (0,2 Mol) N-Acrylyl-N'-(2-chloräthyl)-harnstoff mit 0,2 g, 2,6-Di-tert.-butyl-4-methylphenol in 400 ml Aceton auf 0°C und fügt 23,6 g (0,4 Mol) flüssiges Trimethylamin zu. Man erhitzt in einem Autoklaven 20 Stunden auf 80°C, kühlt ab und filtriert den ausgefallenen Niederschlag. Nach Waschen mit Aceton und Äther erhält man 33 g (0,14 Mol = 70% d. Th.) weißes Kristallpulver.

$C_9H_{18}ClN_3O_2$ 235,71

| | | |
|---|---|---|
| ber. | Cl 15,04 | N 17,83 |
| gef. | Cl 14,90 | N 17,12 |

$^1$H-NMR-Spektrum (in $D_2O$):
$\delta = 3{,}25$ (s, 9); 3,5—4,0 (m, 4); 5,8—6,5 (m, 3)

Der N-(2-Chloräthyl)-N'-acrylylharnstoff wird wie folgt hergestellt:
Ein Gemisch aus 71,1 g (1,0 Mol) Acrylamid, 0,5 g Triäthylamin, 0,5 g 2,6 Di-tert.-butyl-4-methyl-phenol und 250 ml Benzol wird mit 110,8 g (1,05 Mol) 2-Chloräthylisocyanat versetzt und 10 Stunden in einem Autoklaven auf 110°C erhitzt. Der beim Abkühlen ausfallende feinkristalline Niederschlag wird abgefiltert. Man erhält 140 g Substanz, die aus 700 ml Acetonnitril umkristallisiert wird, wobei 115 g (0,65 Mol = 65% d. Th.) weiße Kristallnadeln mit Fp = 156—158°C resultieren.

$C_6H_9ClN_2O_2$ 176,60

| | | |
|---|---|---|
| ber. | Cl 20,07 | N 15,86 |
| gef. | Cl 19,80 | N 15,90 |

$^1$H-NMR-Spektrum (in $CDCl_3$):
$\delta = 3{,}5$—3,8 (m, 4); 5,7—6,8 (m, 3); 9,05 (m, 1); 10,4 (m, 1)

### Beispiel 2

### Methacrylyl-ureylen-äthylen-trimethyl-ammoniumchlorid

Nach der Arbeitsweise von Beispiel 1 erhält man aus 38,1 g (0,2 Mol) N-(2-Chloräthyl)-N'-methacrylylharnstoff 34 g (0,14 Mol = 68% d. Th.) weißes Kristallpulver.

$C_{10}H_{20}ClN_3O_2$ 249,74
ber. Cl 14,20 N 16,83
gef. Cl 14,56 N 16,76

$^1H$-NMR-Spektrum (in $D_2O$):
$\delta$=1,95 (d, 3); 3,2 (s, 9); 3,5—4,0 (m, 4); 5,6—5,9 (m, 2)

## Beispiel 3

### Acrylyl-ureylen-äthylentrimethylammoniumjodid

Man kühlt 26,8 g (0,1 Mol) N-Acrylyl-N'-(2-jodäthyl)-harnstoff mit 0,2 g 2,6-Di-tert.-butyl-4-methylphenol in 300 ml Aceton auf 0°C und fügt 11,8 g (0,2 Mol) flüssiges Trimethylamin zu. Man rührt 24 Stunden und filtriert den entstandenen Niederschlag ab. Nach Waschen mit Aceton und Äther erhält man 30 g (0,092 Mol=92% d. Th.) weißes Kristallpulver.

$C_9H_{18}JN_3O_2$ 327,17
ber. J 38,79 N 12,84
gef. J 39,24 N 12,55

$^1H$-NMR-Spektrum (in $D_2O$):
$\delta$=3,3 (s, 9); 3,6,4,0 (m, 4); 5,9—6,7 (m, 3)

## B) Herstellung und Anwendung der Polymerisate

## Beispiele 4 bis 8

### Copolymerisate aus Acrylamid und erfindungsgemäßen Monomeren (allgemeine Vorschrift)

Man löst das Acrylamid sowie das kationische Monomere in Wasser und stellt die Temperatur der Lösung auf 15°C und den pH-Wert auf 3,0 ein. Die Lösung wird durch Einblasen von Stickstoff von Sauerstoff befreit (1 Stunde). Man katalysiert mit den in Tabelle 1 angegebenen Mengen 2,2'Azo-bisisobutyronitril (AIBN), Kaliumperoxodisulfat sowie Natriumdithionit und Eisen(II)-sulfat, wonach die Polymerisation sofort anläuft. Die Maximaltemperatur wird nach etwa 30 bis 60 Minuten erreicht. Man läßt abkühlen, zerkleinert das entstandene farblose Gel und entwässert es mit 2 bis 3 l Methanol. Nach Filtration wird anhaftendes Methanol im Vakuum abgezogen. Die Ausbeite ist etwa quantitativ.

Tabelle 1

| Beisp. | ACA (g) | QUAT Menge (g) | n. Beisp. | Wasser (g) | WS % | KATALYSATOR AIBN (mg) | $K_2S_2O_8$ (mg) | $Na_2S_2O_4/FeSO_4$ (mg) | Temp. (°C) | Visk. d. d. 1%ig. Lösung (mPa · sec) | Mol-Gew. viskosim. gem. |
|---|---|---|---|---|---|---|---|---|---|---|---|
| 4 | 90 | 10 | 1 | 300 | 25 | 5 | 3 | 10/2 | 80 | 920 | $2{,}2 \times 10^6$ |
| 5 | 70 | 30 | 1 | 300 | 25 | 10 | 6 | 20/4 | 70 | 720 | $1{,}6 \times 10^6$ |
| 6 | 90 | 10 | 2 | 300 | 25 | 5 | 3 | 10/2 | 78 | 2000 | $2{,}7 \times 10^6$ |
| 7 | 70 | 30 | 2 | 300 | 25 | 10 | 4 | 15/2 | 77 | 1040 | $1{,}5 \times 10^6$ |
| 8 | 180 | 20 | 3 | 600 | 25 | 10 | 5 | 15/2 | 70 | 500 | $1{,}8 \times 10^6$ |

ACA = Acrylamid
QUAT = quartäre (Meth)acrylylharnstoffe
WS = wirksame Substanz
AIBN = 2,2'-Azo-bis-isobutyronitril
Temp. = Temperatur
Visk. = Viskosität
gem. = gemessen

## Beispiel 9

Die Anwendbarkeit der erfindungsgemäßen Polymerisate als Entwässerungsmittel, z. B. für Kommunalschlämme, erläutern die nachfolgend beschriebenen beiden Untersuchungen. Die Entwässerungszeiten wurden dabei nach der CST (capillary suction time)-Methode*) festgestellt.

Tabelle 2

| Beispiel | Viskosität der 1%ig. Lösung in dest. Wasser (mPa · sec) | Kapillare Fließzeit bei Zugabe einer 0,1%igen Lösung in Krefelder Leitungswasser ($t_F$ sec) | | | |
|---|---|---|---|---|---|
| | | Zugabe 80 | 100 | 125 | 150 ppm |
| 4 | 920 | 71 | 45 | — | 21 |
| 5 | 720 | — | 50 | 40 | 11 |
| Produkt auf Basis **)DMAEMA · $CH_3Cl$ | 9000 | — | — | 47 | 30 |
| | | Zugabe 100 | 150 | 200 | |
| 6 | 2000 | 127 | 53 | 33 | |
| 7 | 1040 | 74 | 32 | 14 | |
| Produkt auf Basis **)DMAEMA · $CH_3Cl$ | 9000 | 200 | 63 | 22 | |

*) Literatur:
1) R. Baskerville u. R. Gale
A simple automatic instrument for determinating the filtrability of sewage sludges
Water Pollution Control 67 (1968), 233—241
2) R. Gale u. R. Baskerville
Capillary Suction method for determination of the filtration properties of a solid/liquid suspension
Chemistry & Industry (1967), 355—356

**) DMAEMA = 2-Dimethylamino-ethylmethacrylat

Trotz niedriger Viskosität der wäßrigen Lösungen bringen die Produkte durchweg bessere Werte als die bisher angewendeten Copolymerisate auf Basis DMAEMA · $CH_3Cl$.

## Beispiel 10

Die Anwendbarkeit der Produkte als Entwässerungs- und Retentionshilfsmittel bei der Papierherstellung erläutern die nachfolgend beschriebenen Unterschungen, die an standardisierten Zellstoffsuspensionen durchgeführt wurden.

| | |
|---|---|
| Papierstoff: | 60% Fichte-Sulfit<br>40% Laubholzzellstoff |
| Füllstoff: | 30% SPS-Clay<br>(Spezial-China-Clay der Firma English China Clay Co., Ltd. St. Austel, Cornwall — GB) |
| Mahlgrad: | 38° SR |
| Dosierung des Hilfsmittels: | 0,02% bezogen auf Papierstoff |

Zur Beurteilung der Wirksamkeit der Produkte wurden die Entwässerungszeiten und der Retentionseffekt in einem Schopper-Riegler-Mahlgradprüfer ermittelt. Die Ergebnisse sind in Tabelle 3 zusammengefaßt:

Tabelle 3

| Produkt | Entwässerungszeit (sec) | Retention (%) |
|---|---|---|
| Blindprobe | 25 | 72 |
| Beispiel 4 | 21 | 90 |
| Beispiel 5 | 18 | 89 |
| Copolymerisat auf Basis DMAEMA · $CH_3Cl$ | 29 | 91 |

DMAEMA = 2-(Dimethylamino)-ethylmethacrylat

Es zeigt sich, daß die erfindungsgemäß hergestellten Produkte unter den Versuchsbedingungen, die weitgehend den Untersuchungsmethoden der Papiertechnologie entsprechen, wirksam sind. Da die Produkte in wäßriger Lösung zudem leichtflüssig und daher einfach zu handhaben, d. h. leicht dosierbar und leicht löslich sind, können sie bei der Papierherstellung bevorzugt verwendet werden.

Beispiel 11

Die erfindungsgemäß hergestellten Produkte wurden als Sedimentationsmittel in Flockungsversuchen getestet. Durch Bestimmung der Sedimentationzeit wurde das Flockungsverhalten in wäßriger Lösung nach Zusatz zu wäßrigen Tonsuspensionen, die durch Aufschlämmen von Kaolin in Wasser hergestellt worden waren und mit Aluminiumsulfatlösung auf einen pH-Wert von etwa 4,8 eingestellt wurden, geprüft. Die Ergebnisse sind in Tabelle 4 dargestellt.

Tabelle 4

Flockungseffekt auf eine Tontrübe mit 20 g/l Feststoffgehalt:

| Produkt | Zusatzmenge (ppm) | Zeit (sec) |
|---|---|---|
| Blindprobe | | 180 |
| Beispiel 4 | 4 | 4,7 |
| Beispiel 5 | 4 | 14,0 |
| Beispiel 6 | 4 | 6,9 |
| Beispiel 7 | 4 | 18,6 |
| Beispiel 8 | 4 | 5,4 |

Literatur:
H. Akyel und M. Neven, Chemie-Ing. Technik 39 (1967), 172.

**Patentansprüche für die Vertragsstaaten: BE, CH, DE, FR, GB, IT, LI, LU, NL, SE**

1. Polymerisate von quartäre Gruppen enthaltenden Acrylyl- oder Methacrylylharnstoffen, gekennzeichnet durch kleinste wiederkehrende Einheiten der Formel

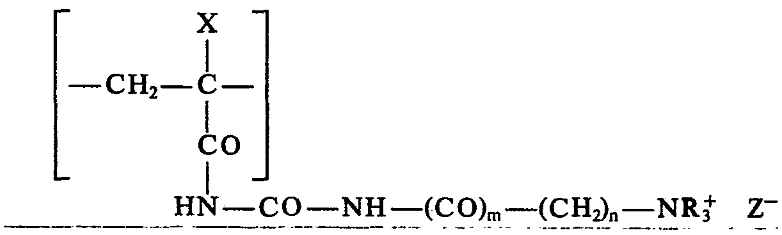

worin X entweder Wasserstoff oder eine Methylgruppe, R ein niederer Alkylrest mit 1 bis 4 Kohlenstoffatomen, m entweder 0 oder 1 und n eine ganze Zahl zwischen 1 und 4 bedeutet und Z für ein beliebiges salzbildendes Anion, vorzugsweise ein Halogenidion steht.

2. Copolymerisate nach Anspruch 1, dadurch gekennzeichnet, daß sie zu 4 bis 96 Gew.-% aus wiederkehrenden Einheiten nach Anspruch 1 und zu 96 bis 4% aus copolymerisierten Vinylmonomeren bestehen.

3. Verwendung der Polymerisate nach Ansprüchen 1 bis 2 als Flockungs-, Sedimentations-, Entwässerungs- und Retentionshilfsmittel.

**Patentansprüche für den Vertragsstaat: AT**

1. Verfahren zur Herstellung von Homo- und Copolymerisaten, die wiederkehrende Einheiten der Formel

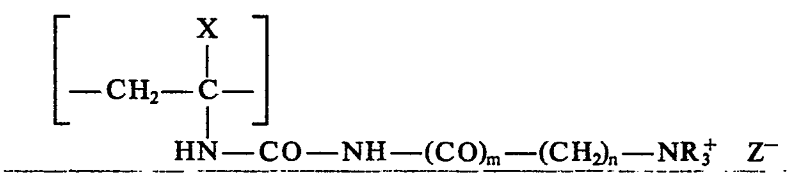

aufweisen, worin X entweder Wasserstoff oder eine Methylgruppe, R ein niederer Alkylrest mit 1 bis 4 Kohlenstoffatomen ist, m entweder 0 oder 1 und n eine ganze Zahl zwischen 1 und 4 bedeuten und Z für ein beliebiges salzbildendes Anion, wie Chlorid, Bromid oder Jodid steht und wobei entsprechende Copolymere 4 bis 96 Gew.-% der vorstehend genannten wiederkehrenden Einheiten enthalten und der Rest aus einem oder mehreren damit copolymerisierbaren Vinylmonomeren besteht, dadurch gekennzeichnet, daß man ungesättigte Ausgangsverbindungen der Formel

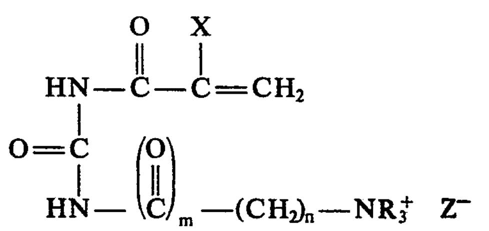

(I)

worin X, R, m, n und $Z^-$ die obige Bedeutung haben, allein oder mit anderen polymerisierbaren Monomeren polymerisiert.

2. Verfahren nach Anspruch 1, dadurch gekennzeichnet, daß man die ungesättigten monomeren Harnstoffe der Formel I mit wasserlöslichen, olefinisch ungesättigten Monomeren copolymerisiert.

3. Verfahren nach Anspruch 2, dadurch gekennzeichnet, daß man als wasserlösliche, olefnisch ungesättigte Comonomere Acrylsäureamid, Methacrylsäureamid, die N-substituierten Derivate von Acryl- oder Methacrylsäureamid, insbesondere N-[3-Dimethylamino-propyl]-acrylamid, die Salze und quartären Verbindungen dieser N-substituierten Derivate, N-Methylolacrylamid, N-Methylolmethacrylamid, die Ether von N-Methylolacrylamid oder -methacrylamid, aminosubstituierte Acryl- oder Methacrylsäureester einschließlich deren Salzen und quartären Verbindungen oder Vinylester oder ein Gemisch aus wenigstens zwei der vorstehend genannten Verbindungen, verwendet.

4. Verfahren nach Ansprüchen 1 bis 3, dadurch gekennzeichnet, daß die Polymerisation mit radikalischen Initiatoren durchgeführt wird.

5. Verfahren nach Ansprüchen 1 bis 4, dadurch gekennzeichnet, daß die Polymerisation in Lösung durchgeführt wird.

**Claims for Contracting States: BE, CH, DE, FR, GB, IT, LI, LU, NL, SE**

1. Polymers of acrylyl or methacrylyl ureas containing quaternary groups, characterised by smallest repeating units of the formula

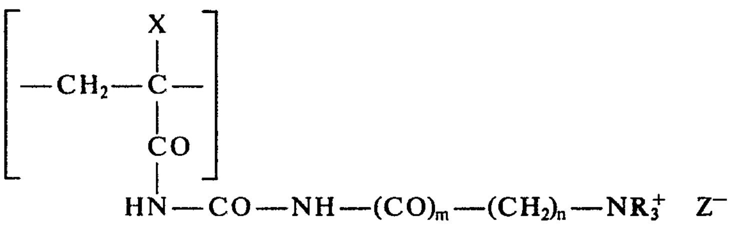

in which X represents either hydrogen or a methyl group, R represents a lower alkyl radical having from 1 to 4 carbon atoms, m represents either 0 or 1 and n represents an integer between 1 and 4, and Z represents any desired salt-forming anion, preferably a halide ion.

2. Copolymers according to claim 1, characterised in that they consist of from 4 to 96% by weight of repeating units according to claim 1 and from 96 to 4% of copolymerised vinyl monomers.

3. Use of the polymers according to claims 1 and 2 as flocculating, sedimentation, dehydrating and retention auxiliaries.

**Claims for Contracting State: AT**

1. Process for the manufacture of homo- and copolymers having the repeating units of the formula

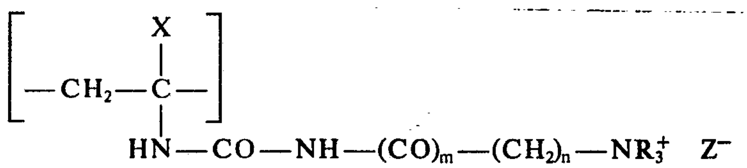

in which X represents either hydrogen or a methyl group, R represents a lower alkyl radical having from 1 to 4 carbon atoms, m represents either 0 or 1 and n represents an integer between 1 and 4, and Z represents any desired salt-forming anion, such as a chloride, bromide or iodide, corresponding copolymers containing from 4 to 96% by weight of the above-metioned repeating units and the remainder consisting of one or more vinyl monomers that can be copolymerised therewith, characterised in that unsaturated starting compounds of the formula

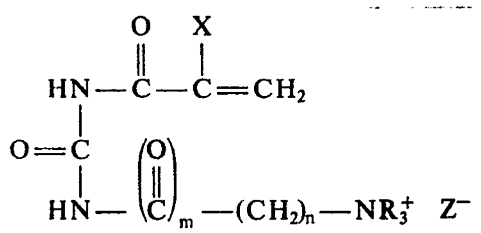

(I)

in which X, R, m, n and $Z^-$ have the above meanings, are polymerised alone or with other polymerisable monomers.

2. Process according to claim 1, characterised in that the unsaturated monomeric ureas of the formula I are copolymerised with water-soluble, olefinically unsaturated monomers.

3. Process according to claim 2, characterised in that there are used as water-soluble, olefinically unsaturated comonoers acrylic acid amide, methacrylic acid amide, N-substituted derivatives of acrylic or methacrylic acid amide, especially N-[3-dimethylaminopropyl]-acrylamide, salts and quaternary compounds of these N-substituted derivatives, N-methylolacrylamide, M-methylolmethacrylamide, ethers of N-methylolacrylamide or N-methylolmethacrylamide, amino-substituted acrylic acid esters or methacrylic acid esters, including their salts and quaternary compounds, or vinyl esters or a mixture of at least two of the above-metioned compounds.

4. Process according to claim 1 to 3, characterised in that the polymerisation is carried out using radical initiators.

5. Process according to claim 1 to 4, characterised in that the polymerisation is carried out in solution.

**Revendications pour les Etats contractants: BE, CH, DE, FR, GB, IT, LU, NL, SE, LI**

1. Polymères d'acrylyl- ou de méthacrylyl-urées contenant des groupes quaternaires, caractérisés en ce qu'ils comprennent des unités récurrentes de la formule

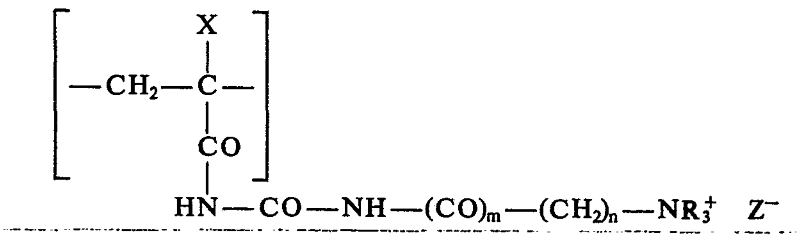

dans laquelle X désigne un atome d'hydrogène ou un radical méthyle et R un radical alcoyle inférieur en $C_1$ à $C_4$, m vaut 0 ou 1 et n un nombre entier de 1 à 4 et Z représente un anion salifiant quelconque, de préférence un ion halogénure.

2. Copolymères suivant la revendication 1, caractérisés en ce qu'ils sont formés de 4 à 96% en poids d'unités récurrentes selon la revendication 1 et de 96 à 4% en poids de monomères vinyliques copolymérisés.

3. Utilisation des polymères suivant les revendications 1 et 2 comme adjuvants de floculation, de sédimentation, de déshydratation et de rétention.

**Revendications pour l'Etat contractant: AT**

1. Procédé de préparation d'homo- et de co-polymères, contenant des unités récurrentes de la formule

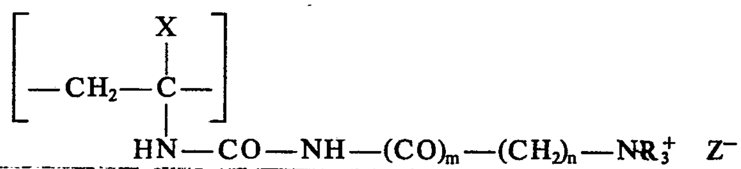

dans laquelle X désigne un atome d'hydrogène ou un radical méthyle et R un radical alcoyle inférieur en $C_1$ à $C_4$, m vaut 0 ou 1, n est un nombre entier de 1 à 4 et $Z^-$ représente un anion salifiant quelconque tel qu'un anion chlorure, bromure ou iodure, les copolymères contennant entre 4 et 96% en poids des unités récurrentes ci-dessus, le reste étant constitué d'un ou de plusieurs monomères vinyliques copolymérisables avec celles-ci, caractérisé en ce que l'on polymérise des composés non saturés de la formule

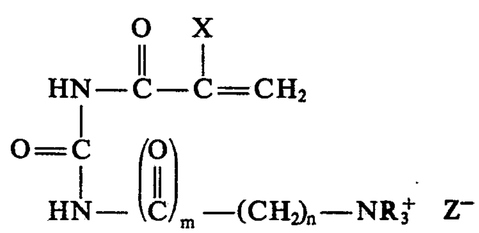

(I)

dans laquelle X, R, M, n est $Z^-$ possèdent la signification définie, soit seuls, soit avec d'autres monomères polymérisables.

2. Procédé suivant la revendication 1, caractérisé en ce que l'on copolymérise les urées non saturées monomères de la formule I avec des monomères à insaturation oléfinique hydrosolubles.

3. Procédé suivant la revendication 2, caractérisé en ce que l'on choisit les comonomères à insaturation oléfinique hydrosolubles parmi l'amide acrylique, l'amide méthacrylique, les dérivés N-substitués de l'amide arylique ou méthacrylique, en particulier l'amide N-[3-diméthylaminio-propyl]-acrylique, les sels et composés quaternaires de ces dérivés N-substitués, l'amide N-méthylol-acrylique et l'amide N-méthylol-méthacrylique, les éthers de l'amide N-méthylol-(méth)acrylique, les esters amino-substitués d'acide acrylique ou méthacrylique et les sels et composés quaternaires de ceux-ci et les esters vinyliques ou que l'on utilise un mélange de deux ou de plus de deux des composés ci-dessus.

4. Procédé suivant les revendications 1 à 3, caractérisé en ce que la polymérisation est effectuée en présence d'amorceurs à radicaux libres.

5. Procédé suivant les revendications 1 à 4, caractérisé en ce que la polymérisation est effectuée en solution.